# EUROPÄISCHE PATENTSCHRIFT

(11) **EP 0 357 162 B1**
(45) Veröffentlichungstag und Bekanntmachung des Hinweises auf die Patenterteilung: **11.08.1993**
(21) Anmeldenummer: 89250015.8
(22) Anmeldetag: 16.08.1989
(51) Int. Cl.: A23C 21/02, A61K 7/48

(54) **Verfahren zur Modifizierung von Molke**
Process for modifying whey
Procédé de modification du petit-lait

(30) Priorität: 17.08.1988 DE 3827833
(43) Veröffentlichungstag der Anmeldung: 07.03.1990
(73) Patentinhaber: LEITHE, Ulrich, D-40593 Düsseldorf (DE)
(72) Erfinder: Meinhard, Horst, D-5419 Herschbach (DE); Leithe, Ulrich, D-4000 Düsseldorf 13 (DE)
(74) Vertreter: Goddar, Heinz J., Dr.

(56) Entgegenhaltungen:
- DE-A- 2 019 235
- DE-B- 2 819 940
- PATENT ABSTRACTS OF JAPAN, Band 8, Nr. 30 (C-209)[1467], 8. Februar 1984; & JP-A-58 192 811 (YAKULT HONSHA K.K.) 10-11-1983
- PATENT ABSTRACTS OF JAPAN, Band 8, Nr. 34 (C-210)[1471], 15. Februar 1984; & JP-A-58 198 409 (YAKULT HONSHA K.K.) 18-11-1983

## Beschreibung

Die Erfindung betrifft ein Verfahren zur Herstellung waschaktiver Substanzen sowie kosmetischer und pharmazeutischer Erzeugnisse auf der Grundlage von mit Hilfe von Milchsäurebakterien modifizierter Molke.

Aus der DE-A-20 19 235 ist ein Verfahren zur Herstellung waschaktiver Substanzen aus Milch oder Molke bekannt, bei dem neben der Vergärung mittels Milchsäurebaktierien als wesentliche weitere Schritte der Zusatz von Fettalkohol und die Sulfonierung dieses Gemisches vorgeschrieben sind. Ein weiteres Verfahren zur Herstellung waschaktiver Substanzen auf der Grundlage von Molke unter Verwendung von Milchsäurestäbchen ist aus der DE-B-28 19 940 bekannt. Bei diesem Verfahren werden der Molke zuckerhaltige Verbindungen zugesetzt und die so erhaltene Lösung einer dreistufigen Vergärung unterzogen. In beiden Fällen macht die aufwendige Prozeßführung die bekannten Verfahren kosten- und arbeitsintensiv.

Der Erfindung liegt daher die Aufgabe zugrunde, das bekannte Verfahren dahingehend weiterzuentwickeln, daß unter Beibehaltung aller Vorteile eine einfache und kostengünstige Prozeßführung ermöglicht wird und gleichzeitig die Anwendungsbereiche der modifizierten Molke erweitert werden.

Erflndungsgemäß wird diese Aufgabe dadurch gelöst, daß die Molke ohne Zusatz von Fettalkoholen und ohne vorherige andersartige Vergärungsschritte solange einer Vergärung durch Milchsäurebakterien unterworfen wird, bis der ursprünglich in der Molke vorhandene Milchzucker weitgehend abgebaut ist, und daß anschließend die so modifizierte Molke entweder direkt oder nach Zusatz geeigneter Substanzen eingesetzt wird, wobei die Molke nach der Vergärung keiner Sulfonierung unterzogen wird.

In einer Ausführungsform der Erfindung wird dabei die Molke vor oder während der Vergärungs mit mindestens einem Milchsäurebakterienstamm beimpft.

Als besondert vorteilhaft hat es sich herausgestellt, wenn die Milchsäurebakterien aus der Gruppe stammen, die aus Lactobacillus acidophilus, Lactobacillus bulgaris, Lactobacillus lactis, Lactobacillus helveticus, Lactobacillus casei, Streptococcus lactis und Streptococcus thermophilus besteht.

Die Erfindung schlägt weiterhin vor, daß der Molke vor dem Vergärungsschritt Heilkräuter oder pflanzliche Bestandteile oder Auszüge aus diesen zugesetzt werden.

Überraschenderweise beeinträchtigt die erfindungsgemäße, drastische Vereinfachung des bekannten Verfahrens in keiner Weise die mit diesem erzielten besonderen Vorteile, so daß die Verwendbarkeit, Handhabung, Lagerung und Einsetzbarkeit der Molke garantiert ist, ohne deren biologischer Axtivität sowie deren physiologischen und qualitativen Vorteile als wertvoller Rohstoff zu zerstören. Die Qualität des erfindungsgemäß hergestellten Produktes ist dabei den nach dem bekannten dreistufigen Verfahren hergestellten Erzeugnissen in jeder Hinsicht zumindest gleichwertig und kann daher in allen dort genannten Anwendungsformen eingesetzt werden. Zusätzlich ermöglicht das erfindungsgemäße Verfahren auch den Einsatz der modifizierten Molke in weiteren Anwendungsgebieten, wie der Kosmetik und der Pharmazie. Dabei spielt es eine wesentliche Rolle, daß die Kombination der Wirkstoffbestandteile der erfindungsgemäß hergestellten Molke und damit auch die der damit herzustellenden Endprodukte durch die Parameter Bakterienart, Steuerung der Vergärungstemperatur und Dauer des Gärungsprozesses bestimmt wird. So ergeben sich für unterschiedliche spezifizierte Einsatzzwecke jeweils unterschiedliche, molkebasierte Wirkstoffkombinationen mit ihren jeweils besonders vorteilhaften Wirkungsmechanismen.

Zum Einsatz von Molke für verschiedene Einsatzzwecke ist es notwendig, die Molke durch Vergärung mittels geeigneter Bakterien mehr oder weniger von Milchzucker zu befreien und ein natürliches, spezielles Milieu zum Einsatz der Molke zu schaffen. Diese Leistung wird von verschiedenartigen Milchsäurebakterien, die nicht auf die hier konkreten benannten beschränkt sein müssen, erbracht. Dabei kann es sich um Bakterien handeln, die bereits aus der Käseproduktion in der Molke vorhanden sind, wie z.B. Lactobacillus casei und/oder Streptococcus thermophilus, die durch geeignete Steuerung der Vergärungstemperatur ohne weiteren Zusatz frischer Bakterienkultur ausgenutzt werden können. Darüber hinaus wird es aber für spezielle Einsatzzwecke notwending sein, die Molke vor oder während des Vergärungsschritts mit dem (den) für den jeweiligen Einsatzzweck geeignetsten Bakterienstamm (stämmen) zu beimpfen. Hier sind unterschiedlichste Kombinationen möglich. So kann beispielsweise der Start der Vergärung den bereits in der Molke vorhandenen Milchsäurebakterien, wie etwa Steptococcus thermophilus, überlassen bleiben oder durch Beimpfen mit einem entsprechenden Bakterienstamm erreicht werden. Die dadurch erzielte Milieuänderung in der Molke schafft dann u.U. optimale Wachstumsbedingungen für weitere Milchsäurebakterien, z.B. Laktobazillen, die zur Erreichung des gewünschten Produktmilieus benötigt werden und daher zu einem späteren Zeitpunkt der Vergärung zugesetzt werden.

Je nach Einsatzzweck können hierfür homofermentative und/oder heterofermentative Milchsäurebakterien eingesetzt werden, die jeweils unterschiedliche Produktmilieus ausbilden, die speziellen Milieubedingungen und -anforderungen gerecht werden.

Durch den Einsatz und die Verwendung von homofermentativen Milchsäurebakterien wird vorwiegend L(+)-, D(-)- oder DL-Milchsäure gebildet, wodurch ein Milieu entsteht, das besonders haut-, schleimhaut- und körperfreundlich ist. Ein Beispiel für eine derartige homofermentative Milchsäurebakteriengattung ist Lactobacillus acidophilus.

Durch den Einsatz und die Verwendung von heterofermentativen Milchsäurebakterien, wie z.B. Lactobacillus lactis, werden neben Milchsäure auch Essigsäure und andere Stoffwechselprodukte (Aromastoffe, Enzyme, Zitronensäure) gebildet, die ein solches Milieu insbesondere für den Einsatz zur Herstellung waschaktiver Substanzen geeignet machen.

Alle Milchsäurebakterien einschließlich Milchsäurestreptococcen führen aber durch den Abbau von Milchzucker, die Bildung von Milchsäure und die Ausscheidung von CO² zu einem stabilen milchsauren Milieu, das für die genannten und im weiteren noch spezifizierten Anwendungsbereiche geeignet ist. Eine spezielle Wirkstoffcharakteristik kann darüber hinaus auch noch durch die Steuerung der Vergärungstemperatur und die Dauer des Gärungsprozesses erreicht werden.

Das erfindungsgemäße Verfahren wird in geeigneten Behältern oder Tankanlagen bei einer für die jeweilige Milchsäurebakteriengattung optimalen Prozeßtemperatur durchgeführt. Diese liegt beispielsweise bei Lactobacillus acidophilus bei etwa 37° C, kann aber bei anderen Bakterienstämmen nach oben oder unten variieren.

Nach Erreichen der optimalen Prozeßtemperatur, wozu ggf. gekühlt oder beheizt werden muß, wird die Molke dann der Vergärung unterzogen. Der entsprechende Vergärungsprozeß wird bei Erreichen des erwünschten Grads an Milchzuckerabbau abgebrochen. Der vollständige Abbau des Milchzuckers läßt sich durch die gleichzeitige Ausscheidung von Kohlendioxid leicht an der Beendigung der Gasentwicklung feststellen.

Die vergorene Molke oder daraus hergestellte Erzeugnisse können zur besseren Lagerung oder Weiterverarbeitung durch Trocknung, Vakuumtrocknung oder Gefriertrocknung eingeengt oder pulverisiert werden. So kann die nach dem erfindungsgemäßen Verfahren hergestellte Molke beispielsweise durch Vakuumtrocknung auf ca. 1/10 des Ausgangsgewichts eingeengt werden und stellt sich dann als zähflüssige Masse dar, die als Vorprodukt weiterverarbeitet werden kann und damit höhere Wirkstoffkonzentration an vorhandenen Aminosäuren und anderen wertbestimmenden Inhaltsstoffen im Endprodukt ermöglicht.

Insbesondere in den Fällen, in denen eine entsprechende Vorkonzentration des Produktes nicht vorgenommen werden soll, empfiehlt sich eine Verpackung und Lagerung unter CO²-Überschuß, da es sich hierbei um milieugerechte Bedingungen handelt.

Die nach dem erfindungsgemäßen Verfahren hergestellte Molke besitzt ein kontrolliertes stabiles milchsaures biologisches Milieu und ist nach vollständiger Vergärung frei von für manche Anwendungsszwecke problematischem Milchzucker. Sie ist als Produkt vielseitig verwendbar, was detailliert durch die folgenden Beispiele belegt wird.

### Ausführungsbeipiel

Bei der Käseproduktion anfallende Molke wird unverzüglich in Gärtanks verfüllt. Die Temperatur der Molke beträgt ca. 30° C. Der pH-Wert der frischen Molke beträgt 5,8 und der Milchzuckeranteil 4,7 %. Die Molke wird zunächst mit einem aus Reinkultur Lactobacillus acidophilus angeimpften Substrat versetzt. Die Tanks werden dann luftdicht mit Gäraufsätzen verschlossen und eine Vergärtemperatur von 30° C beibehalten. Die Vergärdauer beträgt 25 Tage.

Das so entstandene Molkemodifikat hat nach 25 Tagen einen pH-Wert von 3,4 und einen Milchzuckergehalt von 0,35 % und besitzt ein ausgeprägtes milchsaures Milieu mit einer starken Population Milchsäurebakterien Lactobacillus acidophilus. Dieses Molkemodifikat wird dann bei der Produktion von Körperreinigungsprodukten eingesetzt.

### Verwendungsbeispiel 1

Die nach dem erfindungsgemäßen Verfahren mit Lactobacillus lactis oder Milchsäurestreptococcen, wie Streptococcus lactis und Streptococcus thermophilus, hergestellte Molke kann direkt als saures Reinigungsprodukt oder als Ausgangsmaterial für die Herstellung von Reinigungsmitteln, wie z.B. Spülmitteln, Allzweckreinigern, Haushalts- und Industriereinigern sowie Waschmittel verwendet werden. Wird die modifizierte Molke mit oberflächenaktiven Substanzen wie z.B. Fettalkoholsulfaten, Fettalkoholethersulfaten, Fettalkoholpolyglykolethern auf der Basis linearer, pflanzlicher Fette und Öle, Eiweißfettsäurekondensaten, Coco-Betainen usw. versetzt, so entstehen besonders waschaktive Komplexe. Die so entstandenen milieugerechten waschaktiven Substanzen sind sowohl den biologischen Bedürfnissen des Menschen als auch denen der Umwelt besonders gut angepaßt. So hat sich gezeigt, daß die Verfahrensprodukte keine allergischen Reaktionen beim Kontakt mit menschlicher Haut hervorrufen, toxikologisch günstige Werte aufweisen und keinerlei bakterienhemmende Eigenschaften bezüglich der Abwasserbakterien in der Kläranlage zeigen. Bei Einsatz der erwähnten oberflächenaktiven Substanzen sind die Verfahrensprodukte darüber hinaus auch vollständig biologisch abbaubar und zwar in bedeutend kürzeren Zeiträumen, als dies üblicherweise der Fall ist. Weitere mögliche Zusätze bei der Herstellung von Reinigungs- und Waschmitteln sind Öle, Fette und Wachse, mineralische Hilfsstoffe sowie Kräuter oder Kräuterauszüge.

### Verwendungsbeispiel 2

In Verbindung mit geeigneten oberflächenaktiven Substanzen, wie z.B. Fettalkoholsulfaten, Fettalkoholethersulfaten, Fettalkoholpolyglykolethern auf der Basis linearer, pflanzlicher Fette und Öle, Eiweißfettsäurekondensaten, Coco-Betainen usw. sowie Ölen und Fetten zur Rückfettung kann die nach dem erfindungsgemäßen Verfahren hergestellte Molke auch als Körperreinigungsmittel, z.B. in Form von Waschlotionen, Duschbädern, Schaumbädern usw., eingesetzt werden. Für diesen Einsatzzweck eignet sich insbesondere die Verwendung von Lactobacillus acidophilus für die Modifizierung der Molke, da hierdurch ein besonders haut-, schleimhaut- und körperfreundliches Milieu gebildet wird. Bei entsprechender Einstellung des pH-Wertes weist dieser natürliche Molkekomplex mit seinen Proteinen, Vitaminen, Aminosäuren und Spurenelementen die optimale Übereinstimmung mit dem natürlichen Hautsäureschutz der menschlichen Haut auf.

### Verwendungsbeispiel 3

Die nach dem erfindungsgemäßen Verfahren hergestellte Molke wird als wässrige Basis zur Herstellung von Cremes, Lotionen, Salben oder Masken verwendet, indem man mineralische, tierische und/oder pflanzliche Fette oder Öle mit einem Emulgator vermischt und in das erhitzte Gemisch die modifizierte Molke einarbeitet. Ebensogut ist eine Verarbeitung ohne Erhitzen durch Einsatz eines entsprechenden Kaltemulgators möglich. Die entsprechenden Emulsionen können beliebig parfümiert, mit zusätzlichen Wirkstoffen versehen und, wenn nötig und gewünscht, konserviert werden.

Es hat sich gezeigt, daß die erfindungsgemäß hergestellten Produkte bei Einsatz der geeigneten Milchsäurebakteriengattungen, wie z.B. Lactobacillus acidophilus, durch ihre besondere Affinität zur menschlichen Haut für die Körperpflege besonders geeignet sind. Sie schützen und unterstützen durch den biologisch hergestellten Molkekomplex das natürliche Hautmilieu und damit die biologischen Schutzmechanismen der Haut. Die auf der Basis der erfindungsgemäß hergestellten Molke hergestellten Produkte besitzen, wie die modifizierte Molke selbst, mindestens 60 % der natürlichen Feuchthaltefaktoren der menschlichen Haut in Form von Lactaten, Proteinen, Spurenelementen und Aminosäuren. Die unter Verwendung der modifizierten Molke hergestellten kosmetischen Produkte besitzen darüber hinaus ein natürliches Milchsäuremilieu, das der menschlichen Haut nahe verwandt ist.

### Verwendungsbeispiel 4

Die nach dem erfindungsgemäßen Verfahren hergestellte Molke kann auch zur Herstellung von pharmazeutischen Präparaten zur Verbesserung, Erhaltung und/oder Wiederherstellung des Immunsystems der menschlichen Haut und Schleimhaut Verwendung finden. Das in der erfindungsgemäß hergestellten Molke vorhandene Milieu, das durch den Einsatz von Lactobacillus acidophilus erfolgt und kontrolliert wird, ist beispielsweise mit dem natürlichen Milchsäuremilieu in der weiblichen Scheidenflora identisch und gewährleistet dort einen entzündungs- und infektionshemmenden Schutz.

Es hat sich gezeigt, daß mit der erfindungsgemäß modifizierten Molke oder damit hergestellter Präparate eine Verbesserung von entzündlichen Krankheitszuständen, wie z.B. Fluor albus, durch Wiederherstellung des natürlichen Milchsäuremilieus erreicht werden kann. Auch entzündete Hautstellen können durch Aufbringen von erfindungsgemäß modifizierter Molke günstig beeinflußt werden. Diese Wirkungen können durch den Zusatz von pflanzlichen entzündungshemmenden Wirkstoffen, wie z.B. Kamille, noch verbessert werden. Auch als medizinischer Badezusatz kann die erfindungsgemäß modifizierte Molke allein oder in Kombination mit z.B. Kamille verwendet werden.

### Verwendungsbeispiel 5

Vor der Beimpfung mit Milchsäurebakterien werden der Molke Kamillenblüten zugesetzt. Die gesamte Mischung wird dann der aneroben Vergärung unterzogen. Ein so hergestellter Gärungsauszug von Molke und Kamillenblüten ist Tinktur gegen Hautentzündungen und entzündliche Hautzustände hoch wirksam. Die besondere Wirkung von Heilkräutern, wie in diesem Falle Kamillenblüten, wird offensichtlich durch die gleichzeitige anerobe Vergärung mit der Molke gesteigert und durch die biologische Aktivität und die physiologischen Eigenschaften der vergorenen Molke ergänzt und optimiert.

## Patentansprüche

1. Verfahren zur Herstellung waschaktiver Substanzen sowie kosmetischer und pharmazeutischer Erzeugnisse auf der Grundlage von mit Hilfe von Milchsäurebakterien modifizierter Molke, dadurch gekennzeichnet, daß die Molke ohne Zusatz von Fettalkoholen und ohne vorherige andersartige Vergärungsschritte solange einer Vergärung durch Milchsäurebakterien unterworfen wird, bis der ursprünglich in der Molke vorhandene Milchzucker weitgehend abgebaut ist, und daß anschließend die so modifizerte Molke entweder direkt oder nach Zusatz geeigneter Substanzen eingesetzt wird, wobei die Molke nach der Vergärung keiner Sulfonierung unterzogen wird.

2. Verfahren nach Anspruch 1, dadurch gekennzeichnet, daß die Molke vor oder während der Vergärung mit mindestens einem Milchsäurebakterienstamm beimpft wird.

3. Verfahren nach einem der vorangehenden Ansprüche, dadurch gekennzeichnet, daß die Milchsäurebakterien aus der Gruppe stammen, die aus Lactobacillus acidophilus, Lactobacillus bulgaris, Lactobacillus lactis, Lactobacillus helveticus, Lactobacillus casei, Streptococcus lactis und Streptococcus thermophilus besteht

4. Verfahren nach einem der vorangehenden Ansprüche, dadurch gekennzeichnet, daß der Molke vor dem Vergärungsschritt Heilkräuter oder pflanzliche Bestandteile oder Auszüge aus diesem zugesetzt werden.

## Claims

1. A method of producing wash-active substances and cosmetic and pharmaceutical products based on whey modified by lactic acid bacteria, characterised in that the whey, without addition of fatty alcohols and without other previous fermentation steps, is subjected to fermentation by lactic acid bacteria until the lactose originally present in the whey has substantially decomposed, after which the modified whey is used either directly or after addition of suitable substances, and the whey is not sulphonated after fermentation.

2. A method according to claim 1, characterised in that the whey is inoculated with at least one strain of lactic acid bacteria before or during fermentation.

3. A method according to any of the preceding claims, characterised in that the lactic acid bacteria originate from the group consisting of Lactobacillus acidophilus, Lactobacillus bulgaris, Lactobacillus lactis, Lactobacillus helveticus, Lactobacillus casei, Streptococcus lactis and Streptococcus thermophilus.

4. A method according to any of the preceding claims, characterised in that medicinal herbs or vegetable constituents or extracts therefrom are added to the whey before the fermentation step.

## Revendications

1. Procédé de fabrication de substances détergentes ainsi que de produits cosmétiques et pharmaceutiques à base de petit-lait modifié au moyen de bactéries lactiques caractérisé par le fait que le petit-lait est soumis, sans addition d'alcools gras et sans stades préalables de fermentation d'un autre type,à une fermentation par bactéries lactiques suffisamment longtemps pour que le lactose initialement présent dans le petit-lait soit largement dégradé et ensuite le petit-lait ainsi modifié est utilisé soit directement soit après addition de substances appropriées, le petit-lait n'étant soumis à aucune sulfonation après la fermentation.

2. Procédé selon la revendication 1, caractérisé par le fait que le petit-lait, avant ou pendant la fermentation, est ensemencé avec au moins une souche de bactéries lactiques.

3. Procédé selon l'une des revendications précédentes, caractérisé par le fait que les bastéries lactiques proviennent du groupe constitué par Lactobacillus acidophilus, Lactobacillus bulgaris, Lactobacillus lactis, Lactobacillus helveticus, Lactobacillus casei, Streptococcus lactis et Streptococcus thermophilus.

4. Procédé selon l'une des revendications précédentes, caractérisé par le fait qu'au petit-lait sont ajoutés, avant le stade fermentation, des herbes médicimales ou des constituants végétaux ou extraits de celles-ci.
